# EUROPEAN PATENT APPLICATION

(11) **EP 0 997 125 A1**
(43) Date of publication of application: **03.05.2000**
(21) Application number: 98120476.1
(22) Date of filing: 29.10.1998
(51) Int. Cl.: A61F 13/42

(54) **Measuring system and measuring sensor for detecting and measuring the presence of faeces in an absorbent article**

(71) Applicant: THE PROCTER & GAMBLE COMPANY, Cincinnati, Ohio 45202 (US)
(72) Inventor: Muscat, Andreas, 65824 Schwalbach (DE)
(74) Representative: Canonici, Jean-Jacques

(57) **Abstract**

The present invention relates to sensors which are used to selectively detect the presence of fecal material. The invention also relates to hygienic articles comprising such sensors. The sensor of the present invention has a response factor of at least 2, when exposed to fecal test material in aqueous solution having a concentration of 1 gram of fecal test material per 1 gram of physiological saline solution.

## Description

### FIELD OF THE INVENTION

The present invention relates to sensors which are used to detect the presence of fecal material. The invention also relates to hygienic articles comprising such sensors.

### BACKGROUND

In many situations, the presence of fecal material can lead to unpleasant experiences. Such situations range from diapering infants and incontinent adult persons to livestock breeding. Usually, the presence of fecal material is detected visually or olfactorily.

On many occasions, however, it is desirable to be alerted of the presence of fecal material without visual or olfactory contact, for example when checking a diaper for the presence of fecal material in a public place. In some instances, it may also be that the presence of fecal material is to be detected from a position where neither visual nor olfactory detection of feces is possible. For instance, fecal material present in a baby diaper while the baby is wearing several layers of clothing in the winter or while the baby is wrapped in a blanket may not be detected until the baby is undressed. It would be advantageous for the caretaker of an unconscious patient to be alerted of the presence of fecal material in the diaper or bed of the patient.

Sensors that detect the presence of body exudates for example in diapers are well known in the art (see for example U.S. patent application No. 4,640,276). However, these sensors rely on the presence of wetness such that the these sensors are unable to distinguish between the presence of urine and the presence of fecal material. The latter is much more harmful to the skin of the wearer since urine is readily absorbent into the diaper whereas the fecal matter mostly remains on the topsheet of the diaper.

Hence it is an object of the present invention to provide a sensor that is capable of selectively detecting the presence of fecal material.

### SUMMARY OF THE INVENTION

The present invention provides a sensor capable of selectively detecting the presence of fecal material which has a response factor of at least 2, when exposed to fecal test material in aqueous solution having a concentration of 1 gram of fecal test material per 1 gram of physiological saline solution. A preferred embodiment of the present invention is a sensor comprising microorganisms which specifically assimilate a substance contained in fecal material, for example skatole. Another aspect of the present invention are hygienic articles comprising such sensors.

### DEFINITIONS

The term "sensor" as used herein refers to a device that is capable of detecting an event or a parameter that is associated with an event. Such a parameter is also referred to as the input signal. A parameter associated with an event is any measurable signal that correlates with the occurrence of an event within the frame of reference of the system. A sensor may comprise an input means at which the input signal is received by the sensor. A sensor may also respond to different input signals. The detection of an event results in an response of the sensor, the output signal of the sensor.

The term "response" as used herein refers to an observable change of a sensor, i.e. a change that can be detected by any physical measurement. For example, a response can be a change in the physical condition of the sensor, a change in the chemical condition of the sensor, or the like.

The term "quantitative response" as used herein refers to a response that has a measurable intensity which is in monotonous correlation with the change of the environment of the sensor.

The term "qualitative response" as used herein refers to a response that is substantially characterized by only two different response levels, e.g. "on" and "off". The response switches from one level to the other based on the input signal being above or below a pre-set threshold.

The term "monotonous correlation" as used herein refers to a response to an input that exhibits a monotonously changing intensity as a reaction to an increased input, i.e. the response intensity either only increases or only decreases as a function of an increasing input. Increase or decrease of the intensity may be interrupted by regions of constant intensity but they must not be reversed.

The term "reversible sensor" as used herein refers to a sensor that returns to its original state when the input signal is discontinued. Hence, a reversible sensor is capable of detecting multiple events.

The term "irreversible sensor" as used herein refers to a sensor that does not return to its original state when the input signal is discontinued. Hence, a reversible sensor is only capable of detecting one event.

The term "output device" as used herein refers to a device that has an input means and an output means. A output device is capable of emitting a human noticeable response at its output means as a reaction to a change of the environment at its input means, in particular to the response of a sensor or of the output means of a transmitter in close proximity of or connected to the input means of the signal device.

The term "human noticeable signal" as used herein refers to a signal that can be noticed by a human with any of his senses. In particular, the signal could be visual, acoustic, olfactory, tactile or it could include a change in temperature.

The term "actuator" as used herein refers to a device that comprises "potential" and a means of transforming that potential to perform or activate a function. The potential of the actuator may comprise either stored or potential energy or stored material. The actuator thus may perform or activate a function by transforming potential energy to another form of energy such as for example heat or kinetic energy or by releasing or delivering a stored material.

The term "fecal test material" as used herein refers to the anal body exudates of a healthy human being living on a diet including animal proteins having an age between 20 and 40 years. In particular, the human being should not suffer from a disorder of the digestive system such as diarrhea. It is to be understood that this definition is not intended to limit the applicability of the present invention. It is believed that the above test material is a reproducible, representative sample of the anal body exudates of mammalian beings of all ages.

The term "test urine" as used herein refers to the urethral body exudates of a healthy human being living on a diet including animal proteins having an age between 20 and 40 years. In particular, the human being should not suffer from disorders of the digestive system, the kidneys or the urogenital system. It is to be understood that this definition is not intended to limit the applicability of the present invention. It is believed that the above test material is a reproducible, representative sample of the urethral body exudates of mammalian beings of all ages.

### DETAILED DESCRIPTION OF THE INVENTION

The present invention relates to sensors that are capable of selectively detecting the presence of fecal material. In particular, the sensor of the present invention is capable of selectively detecting the presence of BM alone and in mixtures with, for example, other body exudates such as urine.

The effectiveness of the sensors of the present invention can be measured with the Response Factor Test described below. The Response Factor describes the ratio of the response of the sensor when exposed to fecal test material compared to the response of the sensor when exposed to physiological saline solution.

The sensor of the present invention has a response factor of at least 2, preferably at least 3, more preferably at least 5, even more preferably at least 10, most preferably at least 20 when exposed to fecal test material in aqueous solution having a concentration of 1 gram of fecal test material per 1 gram of physiological saline solution. Such a sensor is able to clearly distinguish between the presence of fecal material and the presence of physiological saline solution. Physiological saline solution is used here to represent the background input signal which is present in most natural environments such as aqueous body fluids. The background signal, however, may be very small.

In a preferred embodiment of the sensor of the present invention , the ratio of the response factor of said sensor when exposed to a fecal test material in test urine having a concentration of 1 gram of fecal material per 1 gram of test urine to the response factor of said sensor when exposed to test urine is at least 2, preferably at least 3, more preferably at least 5, even more preferably at least 10, most preferably at least 20. This preferred sensor is particularly useful in discriminating between the presence of fecal material and the presence of urine. Such a discrimination is particularly useful in diapering babies or adult incontinent persons where the presence of fecal material may be substantially more harmful to the skin than the presence of urine which is readily absorbed by the diaper.

In one preferred embodiment of the present invention, the sensor is a quantitative sensor, more preferably having a monotonous response. A quantitative sensor is useful in combination with output devices that have a continuous output such as a surface that exhibits a color change from white to black with, preferably multiple, intermediate shades of gray. Such output devices allow the user of the sensor to receive an indication of the concentration of fecal material at the sensor from the output signal of the signaling device. A typical application of such a sensor would be a device to control the soiling of the floor of a stable, cow-shed, pigsty, or the like. Based on the continuous output signal of the quantitative sensor, the time until the floor is to be cleaned can be estimated. Such a sensor may also be deployed to show the filling level of camping toilets which collect the exudates in a reservoir.

Further, a sensor of the present invention may also be reversible or irreversible. For some applications is it sufficient if the sensor is irreversible, for example when the sensor is used in a disposable article, i.e. article and sensor are disposed of once the article is soiled with fecal material. In other applications it is preferred to have a reversible sensor, for example when multiple events have to be detected.

In another equally preferred embodiment of the present invention, the sensor is a qualitative sensor. A qualitative sensor provides the possibility to connect the sensor to a binary signal device, i.e. a signal device that has only two outputs such as "on" and "off". A qualitative sensor can be obtained by combining a quantitative sensor with a discriminator that converts a continuous input into a binary output signal based on a pre-set threshold. A typical application of a qualitative sensor of the present invention is a baby diaper where the sensor in combination with a binary signal device may be used by the mother to be alerted when its time to change a soiled diaper. Another application of a qualitative sensor of the present invention would be to measure the time of defecation of a mammalian being. In this case, the output of the sensor could trigger a clock.

In a preferred embodiment of the sensor of the present invention, the sensor detects the presence of skatole in fecal material. This preferred sensor device has a response factor of at least 2, preferably at least 3, more preferably at least 5, even more preferably at least 10, most preferably at least 20 when exposed to skatole in an aqueous solution having a concentration of 180 micrograms of skatole per gram of physiological saline solution.

It has been found that the skatole concentration in feces is around 180 microgram per gram of fecal material (A. Sohler and J. Noval, Curr. Conc. Res., pp. 669-677, 1969) whereas the skatole level in urine has been found to be substantially lower (M. T. Dmitriev, Kosm. Biol. Aviakosm. 21(4), pp. 50-56, 1987). Skatole is a microbiological degradation product that originates from the catabolism of tryptophane in the intestinal system. Hence, the detection of skatole is one way to detect the presence of fecal material.

In one preferred embodiment of the present invention, the sensor device comprises microorganisms including genetically engineered microorganisms. Microorganisms can be obtained that assimilate specific substances such as for example skatole. The assimilation of specific substances can be measured, for example, via the oxygen consumption during this process. Hence, such microorganisms may serve as a component of a sensor suitable for the present invention. Suitable sensors based on microorganisms are commercially available for example from Institut für Chemo- und Biosensorik of Münster, Germany, under the designation Mikrobielle Sensoren. Microorganisms having a fast response time are preferred for purposes of the present invention since they enable a faster detection of the fecal material.

Microorganisms suitable for the sensor of the present invention are, for example, Acinetobacter baumannii TOI63 (FERM P-12891, Japanese patent publication JP05304947), Bacillus sp TOI41(FREM P-12914, Japanese patent publication JP05304948).

Microorganisms comprised in a sensor are immobilized in the sensor by techniques known in the art such as entrapment, adsorption, crosslinking, encapsulation, covalent attachment, any combination thereof, or the like (see for example "Entwicklung und Charakterisierung von polymeren Träger und Immobilisatmaterialien für eine verbesserte Sauerstoffversorgung", Papierflieger, Clausthal-Zellerfeld, Germany, 1995). The immobilization can be carried out on many different substrates such as known the art. Preferably, the immobilization substrate is selected from the group of polymer based materials, hydrogels, tissues, nonwoven materials, woven materials. The term "hydrogel" as used herein refers to a polymeric material forming a three-dimensional, water containing matrix comprising less than 50%, preferably less than 30% more preferably less than 20%, most preferably less than 10% by weight of the polymeric material.

It is another aspect of the present invention to provide hygienic articles comprising the sensor capable of selectively detecting the presence of fecal material according to the present invention. The term "hygienic article" as used herein refers to articles which are worn in contact to or in close proximity of the skin of the wearer. Hygienic article include absorbent articles and non-absorbent articles, disposable article and re-usable articles. Typical hygienic articles are diapers, incontinence pads, incontinence briefs, incontinence undergarments, absorbent inserts, diaper holders and liners, fecal material management device to be attached to a natural or artificial anus such as colostomy bags and the like, feminine hygiene garments, wipes, disposable towels, tissues, water absorbing articles, bandages, therapeutic wraps, supports, disposable heating pads, bed pans and the like.

As used herein, the term "absorbent article" refers to devices which absorb and contain body exudates, and more specifically, refers to devices which are placed against or in proximity to the body of the wearer to absorb and contain the various exudates discharged from the body. The term "disposable" is used herein to describe absorbent articles which generally are not intended to be laundered or otherwise restored or reused as an absorbent article (i.e., they are intended to be discarded after a single use and, preferably, to be recycled, composted or otherwise disposed of in an environmentally compatible manner). (As used herein, the term "disposed" is used to mean that an element(s) of the diaper is formed (joined and positioned) in a particular place or position as a unitary structure with other elements of the diaper or as a separate element joined to another element of the diaper. As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.) A "unitary" absorbent article refers to absorbent articles which are formed of separate parts united together to form a coordinated entity so that they do not require separate manipulative parts like a separate holder and liner. A particular embodiment of an absorbent article of the present invention is a unitary disposable absorbent article, such as a diaper. As used herein, the term "diaper" refers to an absorbent article generally worn by infants and incontinent persons about the lower torso.

The diaper 20 preferably comprises a liquid pervious topsheet 24; a liquid impervious backsheet 26; an absorbent core 28, which is preferably positioned between at least a portion of the topsheet 24 and the backsheet 26; side panels 30; elasticized leg cuffs 32; an elastic waist feature 34; and a fastening system generally designated 40. Diaper 20 is shown in Figure 1 to have a first waist region 36, a second waist region 38 opposed to the first waist region 36 and a crotch region 37 located between the first waist region and the second waist region. The periphery of the diaper 20 is defined by the outer edges of the diaper 20 in which the longitudinal edges 50 run generally parallel to the longitudinal centerline 100 of the diaper 20 and the end edges 52 run between the longitudinal edges 50 generally parallel to the lateral centerline 110 of the diaper 20.

The chassis 22 of the diaper 20 comprises the main body of the diaper 20. The chassis 22 comprises at least a portion of the absorbent core 28 and preferably an outer covering layer including the topsheet 24 and the backsheet 26. If the absorbent article comprises a separate holder and a liner, the chassis 22 generally comprises the holder and the liner. (For example, the holder may comprise one or more layers of material to form the outer cover of the article and the liner may comprise an absorbent assembly including a topsheet, a backsheet, and an absorbent core. In such cases, the holder and/or the liner may include a fastening element which is used to hold the liner in place throughout the time of use.) For unitary absorbent articles, the chassis 22 comprises the main structure of the diaper with other features added to form the composite diaper structure. While the topsheet 24, the backsheet 26, and the absorbent core 26 may be assembled in a variety of well known configurations, preferred diaper configurations are described generally in U.S. Pat. No. 3,860,003 entitled "Contractible Side Portions for Disposable Diaper" which issued to Kenneth B. Buell on January 14, 1975; U.S. Pat. No. 5,151,092 issued to Buell on September 9, 1992; and U.S. Pat. No. 5,221,274 issued to Buell on June 22, 1993; and U.S. Pat. No. 5,554,145 entitled "Absorbent Article With Multiple Zone Structural Elastic-Like Film Web Extensible Waist Feature" which issued to Roe et al. on September 10, 1996; U.S. Pat. No. 5,569,234 entitled "Disposable Pull-On Pant" which issued to Buell et al. on October 29, 1996; U.S. Pat. No. 5,580,411 entitled "Zero Scrap Method For Manufacturing Side Panels For Absorbent Articles" which issued to Nease et al. on December 3, 1996; and PCT Patent Application publishes as WO95/13775 entitled "Absorbent Article With Multi-Directional Extensible Side Panels" filed in the name of Robles et al.; each of which is incorporated herein by reference.

The backsheet 26 is generally that portion of the diaper 20 positioned adjacent the garment facing surface 45 of the absorbent core 28 which prevents the exudates absorbed and contained therein from soiling articles which may contact the diaper 20, such as bedsheets and undergarments. In preferred embodiments, the backsheet 26 is impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, IN and sold under the trade names X15306, X10962 and X10964. Other suitable backsheet materials may include breathable materials which permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P18-3097. Such breathable composite materials are described in greater detail in PCT Application No. WO 95/16746, published on June 22, 1995 in the name of E. I. DuPont and copending PCT Patent Application published as WO98/19861, filed on November 6, 1996 in the name of Curro. Other breathable backsheets including nonwoven webs and apertured formed films are described in U.S. Pat. No. 5,571,096 issued to Dobrin et al. on November 5, 1996. Each of these references is hereby incorporated by reference herein.

The backsheet 26, or any portion thereof, may be elastically extensible in one or more directions. In one embodiment, the backsheet 26 may comprise a structural elastic-like film ("SELF") web. A structural elastic-like film web is an extensible material that exhibits an elastic-like behavior in the direction of elongation without the use of added elastic materials. The SELF web includes a strainable network having at least two contiguous, distinct, and dissimilar regions. Preferably, of the regions is configured so that it will exhibit resistive forces in response to an applied axial elongation in a direction parallel to the predetermined axis before a substantial portion of the other region develops significant resistive forces to the applied elongation. At least one of the regions has a surface-pathlength which is greater than that of the other region as measured substantially parallel to the predetermined axis while the material is in an untensioned condition. The region exhibiting the longer surface-pathlength includes one or more deformations which extend beyond the plane of the other region. The SELF web exhibits at least two significantly different stages of controlled resistive force to elongation along at least one predetermined axis when subjected to an applied elongation in a direction parallel to the predetermined axis. The SELF web exhibits first resistive forces to the applied elongation until the elongation of the web is sufficient to cause a substantial portion of the region having the longer surface-pathlength to enter the plane of applied elongation, whereupon the SELF web exhibits second resistive forces to further elongation. The total resistive forces to elongation are higher than the first resistive forces to elongation provided by the first region. SELF webs suitable for the present invention are more completely described in U.S. Patent No. 5,518,801 entitled Web Materials Exhibiting Elastic-Like Behavior, which issued to Chappell, et, al. on May 21, 1996, which is incorporated herein by reference. In alternate embodiments, the backsheet 26 may comprise elastomeric films, foams, strands, or combinations of these or other suitable materials with nonwovens or synthetic films.

The backsheet 26 may be joined to the topsheet 24, the absorbent core 28 or any other element of the diaper 20 by any attachment means known in the art. For example, the attachment means may include a uniform continuous layer of adhesive, a patterned layer of adhesive, or an array of separate lines, spirals, or spots of adhesive. One preferred attachment means comprises an open pattern network of filaments of adhesive as disclosed in U.S. Patent 4,573,986 entitled "Disposable Waste-Containment Garment", which issued to Minetola et al. on March 4, 1986. Other suitable attachment means include several lines of adhesive filaments which are swirled into a spiral pattern, as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Each of these patents are incorporated herein by reference. Adhesives which have been found to be satisfactory are manufactured by H. B. Fuller Company of St. Paul, Minnesota and marketed as HL-1620 and HL-1358-XZP. Alternatively, the attachment means may comprise heat bonds, pressure bonds, ultrasonic bonds, dynamic mechanical bonds, or any other suitable attachment means or combinations of these attachment means as are known in the art.

The topsheet 24 is preferably positioned adjacent the body surface 47 of the absorbent core 28 and may be joined thereto and/or to the backsheet 26 by any attachment means known in the art. Suitable attachment means are described above with respect to means for joining the backsheet 26 to other elements of the diaper 20. In one preferred embodiment of the present invention, the topsheet 24 and the backsheet 26 are joined directly to each other in some locations and are indirectly joined together in other locations by directly joining them to other elements of the diaper 20.

The topsheet 24 is preferably compliant, soft feeling, and non-irritating to the wearer's skin. Further, at least a portion of the topsheet 24 is liquid pervious, permitting liquids to readily penetrate through its thickness. A suitable topsheet 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the topsheets include fibers, the fibers may be spunbond, carded, wet-laid, meltblown, hydroentangled, or otherwise processed as is known in the art. One suitable topsheet 24 comprising a web of staple length polypropylene fibers is manufactured by Veratec, Inc., a Division of International Paper Company, of Walpole, Massachusetts under the designation P-8.

Suitable formed film topsheets are described in U.S. Pat. No. 3,929,135, entitled "Absorptive Structures Having Tapered Capillaries", which issued to Thompson on December 30, 1975; U.S. Pat. No. 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel, et al. on August 3, 1982; U.S. Pat. No. 4,463,045 entitled "Macroscopically Expanded Three-Dimensional Plastic Web Exhibiting Non-Glossy Visible Surface and Cloth-Like Tactile Impression", which issued to Ahr, et al. on July 31, 1984; and U.S. Pat. No. 5,006,394 "Multilayer Polymeric Film" issued to Baird on April 9, 1991. Other suitable topsheets 30 are made in accordance with U.S. Pat. Nos. 4,609,518 and 4,629,643 which issued to Curro et al. on September 2, 1986 and December 16, 1986, respectively, and both of which are incorporated herein by reference. Such formed films are available from The Procter & Gamble Company of Cincinnati, Ohio as "DRI-WEAVE" and from Tredegar Corporation of Terre Haute, Indiana as "CLIFF-T."

Preferably, the topsheet 24 is made of a hydrophobic material or is treated to be hydrophobic in order to isolate the wearer's skin from liquids contained in the absorbent core 28. If the topsheet 24 is made of a hydrophobic material, preferably at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 28. The topsheet 24 can be rendered hydrophilic by treating it with a surfactant or by incorporating a surfactant into the topsheet. Suitable methods for treating the topsheet 24 with a surfactant include spraying the topsheet 24 material with the surfactant and immersing the material into the surfactant. A more detailed discussion of such a treatment and hydrophilicity is contained in U.S. Pat. No. 4,988,344 entitled "Absorbent Articles with Multiple Layer Absorbent Layers" issued to Reising, et al. on Jan. 29, 1991 and U.S. Pat. No. 4,988,345 entitled "Absorbent Articles with Rapid Acquiring Absorbent Cores" issued to Reising on Jan. 29, 1991. A more detailed discussion of some suitable methods for incorporating surfactant in the topsheet can be found in U.S. Statutory Invention Registration No. H1670, published on July 1, 1997 in the names of Aziz et al. Each of these references is hereby incorporated by reference herein. Alternatively, the topsheet 24 may include an apertured web or film which is hydrophobic. This may be accomplished eliminating the hydrophilizing treatment step from the production process and/or applying a hydrophobic treatment to the topsheet 24, such as a polytetraflouroethylene compound like SCOTCHGUARD or a hydrophobic lotion composition, as described below. In such embodiments, it is preferred that the apertures be large enough to allow the penetration of aqueous fluids like urine without significant resistance.

Any portion of the topsheet 24 may be coated with a lotion as is known in the art. Examples of suitable lotions include those described in U.S. Pat. Nos. 5,607,760 entitled "Disposable Absorbent Article Having A Lotioned Topsheet Containing an Emollient and a Polyol Polyester Immobilizing Agent" which issued to Roe on March 4, 1997; U.S. Pat. No. 5,609,587 entitled "Diaper Having A Lotion Topsheet Comprising A Liquid Polyol Polyester Emollient And An Immobilizing Agent" which issued to Roe on March 11, 1997; U.S. Pat. No. 5,635,191 entitled "Diaper Having A Lotioned Topsheet Containing A Polysiloxane Emollient" which issued to Roe et al. on June 3, 1997; and U.S. Pat. No. 5,643,588 entitled "Diaper Having A Lotioned Topsheet" which issued to Roe et al. on July 1, 1997. The lotion may function alone or in combination with another agent as the hydrophobizing treatment described above. Further, the topsheet 24, the backsheet 26 or any portion of the topsheet or backsheet may be embossed and/or matte finished to provide a more cloth like appearance.

The absorbent core 28 may comprise any absorbent material which is generally compressible, conformable, non-irritating to the wearer's skin, and capable of absorbing and retaining liquids such as urine and other certain body exudates. The absorbent core 28 may be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The configuration and construction of the absorbent core 28 may also be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures). However, the total absorbent capacity of the absorbent core 28 should be compatible with the design loading and the intended use of the diaper 20.

Exemplary absorbent structures for use as the absorbent assemblies are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,834,735, entitled "High Density Absorbent Members Having Lower Density and Lower Basis Weight Acquisition Zones", issued to Alemany et al. on May 30, 1989; U.S. Patent 4,888,231 entitled "Absorbent Core Having A Dusting Layer" issued to Angstadt on December 19, 1989; U.S. Pat. No. 5,137,537 entitled "Absorbent Structure Containing Individualized, Polycarboxylic Acid Crosslinked Wood Pulp Cellulose Fibers" which issued to Herron et al. on August 11, 1992; U.S. Patent 5,147,345 entitled "High Efficiency Absorbent Articles For Incontinence Management" issued to Young et al. on September 15, 1992; U.S. Pat. No. 5,342,338 entitled "Disposable Absorbent Article For Low-Viscosity Fecal Material" issued to Roe on August 30, 1994; U.S. Pat. No. 5,260,345 entitled "Absorbent Foam Materials For Aqueous Body Fluids and Absorbent Articles Containing Such Materials" issued to DesMarais et al. on November 9, 1993; U.S. Pat. No. 5,387,207 entitled "Thin-Until-Wet Absorbent Foam Materials For Aqueous Body Fluids And Process For Making Same" issued to Dyer et al. on February 7, 1995; and U.S. Pat. No. 5,625,222 entitled "Absorbent Foam Materials For Aqueous Fluids Made From high Internal Phase Emulsions Having Very High Water-To-Oil Ratios" issued to DesMarais et al. on July 22, 1997. Each of these patents is incorporated herein by reference.

The diaper 20 may also comprise at least one elastic waist feature 34 that helps to provide improved fit and containment. The elastic waist feature 34 is generally intended to elastically expand and contract to dynamically fit the wearer's waist. The elastic waist feature 34 preferably extends at least longitudinally outwardly from at least one waist edge 62 of the absorbent core 28 and generally forms at least a portion of the end edge 52 of the diaper 20. Disposable diapers are often constructed so as to have two elastic waist features, one positioned in the first waist region 36 and one positioned in the second waist region 38. Further, while the elastic waist feature 34 or any of its constituent elements may comprise one or more separate elements affixed to the diaper 20, the elastic waist feature 34 may be constructed as an extension of other elements of the diaper 20, such as the backsheet 26, the topsheet 24, or both the backsheet 26 and the topsheet 24.

The elastic waist feature 34 may be constructed in a number of different configurations including those described in U.S. Pat. No. 4,515,595 issued to Kievit et al. on May 7, 1985; U.S. Pat. No. 4,710,189 issued to Lash on December 1, 1987; U.S. Pat. No. 5, 151,092 issued to Buell on September 9, 1992; and U.S. Pat. No. 5,221,274 issued to Buell on June 22, 1993. Other suitable waist configurations may include waistcap features such as those described in U.S. Pat. No. 5,026,364 issued to Robertson on June 25, 1991 and U.S. Pat. No. 4,816,025 issued to Foreman on March 28, 1989. All of the above mentioned references are incorporated herein by reference.

The diaper 20 may also include a fastening system 40. The fastening system 40 preferably maintains the first waist region 36 and the second waist region 38 in an overlapping configuration so as to provide lateral tensions about the circumference of the diaper 20 to hold the diaper 20 on the wearer. The fastening system 40 preferably comprises tape tabs and/or hook and loop fastening components, although any other known fastening means are generally acceptable. Some exemplary fastening systems are disclosed in U.S. Patent 3,848,594 entitled "Tape Fastening System for Disposable Diaper" issued to Buell on November 19, 1974; U.S. Patent B1 4,662,875 entitled "Absorbent Article" issued to Hirotsu et al. on May 5, 1987; U.S. Patent 4,846,815 entitled "Disposable Diaper Having An Improved Fastening Device" issued to Scripps on July 11,1989; U.S. Patent 4,894,060 entitled "Disposable Diaper With Improved Hook Fastener Portion" issued to Nestegard on January 16, 1990; U.S. Patent 4,946,527 entitled "Pressure-Sensitive Adhesive Fastener And Method of Making Same" issued to Battrell on August 7, 1990; and the herein before referenced U.S. Pat. No. 5,151,092 issued to Buell on September 9, 1992; and U.S. Pat. No. 5,221,274 issued to Buell on June 22, 1993. The fastening system may also provide a means for holding the article in a disposal configuration as disclosed in U.S. Pat. No. 4,963,140 issued to Robertson et al. on October 16, 1990. Each of these patents is incorporated herein by reference. In alternative embodiments, opposing sides of the garment may be seamed or welded to form a pant. This allows the article to be used as a pull-on type diaper, such as a training pant.

The diaper 20 may also comprise side panels 30. The side panels 30 may be elastic or extensible to provide a more comfortable and contouring fit by initially conformably fitting the diaper 20 to the wearer and sustaining this fit throughout the time of wear well past when the diaper 20 has been loaded with exudates since the elasticized side panels 30 allow the sides of the diaper 20 to expand and contract. The side panels 30 may also provide more effective application of the diaper 20 because even if the diaperer pulls one elasticized side panel 30 farther than the other during application, the diaper 20 will "self-adjust" during wear.

While the diaper 20 of the present invention preferably has the side panels 30 disposed in the second waist region 38, the diaper 20 may be provided with side panels 30 disposed in the first waist region 36 or in both the first waist region 36 and the second waist region 38. The side panels 30 may be constructed in any suitable configurations. Examples of diapers with elasticized side panels are disclosed in U.S. Patent 4,857,067, entitled "Disposable Diaper Having Shirred Ears" issued to Wood, et al. on August 15, 1989; U.S. Patent 4,381,781 issued to Sciaraffa, et al. on May 3, 1983; U.S. Patent 4,938,753 issued to Van Gompel, et al. on July 3, 1990; the herein before referenced U.S. Pat. No. 5,151,092 issued to Buell on September 9, 1992; and U.S. Pat. No. 5, 221,274 issued to Buell on June 22, 1993; U.S. Patent No. 5,669,897 issued to LaVon, et al. on September 23, 1997 entitled "Absorbent Articles Providing Sustained Dynamic Fit"; U.S. Patent Application Serial No. 08/915,471 entitled "Absorbent Article With Multi-Directional Extensible Side Panels" filed August 20, 1997 in the names of Robles, et al.; each of which is incorporated herein by reference.

The diaper 20 preferably further includes leg cuffs 32 which provide improved containment of liquids and other body exudates. Leg cuffs may also be referred to as leg bands, side flaps, barrier cuffs, or elastic cuffs. U.S. Patent 3,860,003 describes a disposable diaper which provides a contractible leg opening having a side flap and one or more elastic members to provide an elasticized leg cuff (a gasketing cuff). U.S. Patent Nos. 4,808,178 and 4,909,803 issued to Aziz et al. on February 28, 1989 and March 20, 1990, respectively, describe disposable diapers having "stand-up" elasticized flaps (barrier cuffs) which improve the containment of the leg regions. U.S. Pat. Nos. 4,695,278 and 4,795,454 issued to Lawson on September 22, 1987 and to Dragoo on January 3, 1989, respectively, describe disposable diapers having dual cuffs, including gasketing cuffs and barrier cuffs. In some embodiments, it may be desirable to treat all or a portion of the leg cuffs with a lotion, as described above.

Embodiments of the present invention may also include pockets for receiving and containing waste, spacers which provide voids for waste, barriers for limiting the movement of waste in the article, compartments or voids which accept and contain waste materials deposited in the diaper, and the like, or any combinations thereof. Examples of pockets and spacers for use in absorbent products are described in U.S. Patent 5,514,121 issued to Roe et al. on May 7, 1996, entitled "Diaper Having Expulsive Spacer"; U.S. Patent 5,171,236 issued to Dreier et al on December 15, 1992, entitled "Disposable Absorbent Article Having Core Spacers"; U.S. Patent 5,397,318 issued to Dreier on March 14, 1995, entitled "Absorbent Article Having A Pocket Cuff"; U.S. Patent 5,540,671 issued to Dreier on July 30, 1996, entitled "Absorbent Article Having A Pocket Cuff With An Apex"; and PCT Application WO 93/25172 published December 3, 1993, entitled "Spacers For Use In Hygienic Absorbent Articles And Disposable Absorbent Articles Having Such Spacer"; and U.S. Patent 5,306,266, entitled "Flexible Spacers For Use In Disposable Absorbent Articles", issued to Freeland on April 26, 1994. Examples of compartments or voids are disclosed in U.S. Patent 4,968,312, entitled "Disposable Fecal Compartmenting Diaper", issued to Khan on November 6, 1990; U.S. Patent 4,990,147, entitled "Absorbent Article With Elastic Liner For Waste Material Isolation", issued to Freeland on February 5, 1991; U.S. Patent 5,62,840, entitled "Disposable Diapers", issued to Holt et al on November 5, 1991; and U.S. Patent 5,269,755 entitled "Trisection Topsheets For Disposable Absorbent Articles And Disposable Absorbent Articles Having Such Trisection Topsheets", issued to Freeland et al on December 14, 1993. Examples of suitable transverse barriers are described in U.S. Pat. No. 5,554,142 entitled "Absorbent Article Having Multiple Effective Height Transverse Partition" issued September 10, 1996 in the name of Dreier et al.; PCT Patent WO 94/14395 entitled "Absorbent Article Having An Upstanding Transverse Partition" published July 7, 1994 in the name of Freeland, et al.; and U.S. 5,653,703 Absorbent Article Having Angular Upstanding Transverse Partition, issued Aug. 5, 1997 to Roe, et al. All of the above-cited references are hereby incorporated by reference herein.

The absorbent article of the present invention comprises a sensor according to the present invention. Preferably, the sensor is joined to or comprised in the topsheet of the absorbent article, more preferably in the back waist region of the absorbent article. The sensor may also be joined to a pocket, a spacer, a barrier, a compartment or void, a barrier cuff, or any other suitable component of the absorbent article.

In one embodiment, the absorbent article may include an actuator that comprises a compressed foam spacer vacuum sealed under a water soluble film (e.g., a PVA film). Upon receipt of the proper signal from the sensor, the actuator closes a switch that releases a small amount of stored water to contact and dissolve the water soluble film. This results in the release of the stored mechanical energy in the compressed foam. The foam expands and forms a spacer to provide void volume for the feces. Alternatively, the switch closure may release two chemicals that combine and create a foaming system, which may engulf the feces.

In another embodiment, the absorbent article may include an output device coupled to the sensor that alerts the caretaker or the wearer of an event such as a defecation. If the output device alerts the caretaker, for example, the caretaker may prepare to change the article to minimize the amount of time that the bodily waste is in contact with the skin of the wearer, may ensure that a bedpan or an absorbent article is in place to contain the bodily waste when it is removed, or may notify the wearer to get to the bathroom in order to remove the bodily waste.

Other suitable output devices and actuators are described in U.S. patent applications having the serial numbers 09/107,561, 09/106,424, 09/107,563, 09/106,225 (P&G cases 7196 through 7199).

### METHODS

### Response Factor Test

With the Response Factor Test as described hereafter the response of a quantitative sensor as a reaction to exposure to a specific substance or composition can be measured.

The specific substances or compositions for which this test is suitable are
- fecal test material in aqueous solution having a concentration of 1 gram of fecal test material per 1 gram of physiological saline solution
- fecal test material in test urine solution having a concentration of 1 gram of fecal material per 1 gram of test urine solution
- test urine solution
- a solution of skatole in physiological saline solution having a concentration of 180 micrograms of skatole per gram of physiological saline solution
- physiological saline solution

All measurements are carried at body temperature (37° Celsius). The method includes the following steps in the following order:
- Record quantitative response of sensor after exposition to physiological saline solution for 24 hours. The background response is the maximum recorded response.
- Expose sensor to specified substance or composition
- Record quantitative response of sensor while sensor is still exposed to the specified substance or composition for 24 hours. Substance response is the maximum recorded response.

The Response Factor is obtained by normalizing the substance response with the background response. In case, the thus obtained Response Factor is smaller than 1, the reciprocal value of the Response Factor is reported as the Response Factor.

## Claims

1. A sensor capable of selectively detecting the presence of fecal material characterized in that said sensor has a response factor of at least 2 when exposed to fecal test material in aqueous solution having a concentration of 1 gram of fecal test material per 1 gram of physiological saline solution according to the Response Factor Test disclosed herein.

2. A sensor capable of selectively detecting the presence of fecal material according to Claim 1 wherein said sensor is a quantitative sensor.

3. A sensor capable of selectively detecting the presence of fecal material according to Claim 1 wherein said sensor is a qualitative sensor.

4. A sensor capable of selectively detecting the presence of fecal material according to Claim 1 wherein the ratio of the response factor of said sensor when exposed to a fecal test material in test urine solution having a concentration of 1 gram of fecal material per 1 gram of test urine solution according to the Response Factor Test disclosed herein to the response factor of said sensor when exposed to test urine solution is at least 2 according to the Response Factor Test disclosed herein.

5. A sensor capable of selectively detecting the presence of fecal material according to Claim 1 wherein said sensor device has a response factor of at least 2 when exposed to a solution of skatole in physiological saline solution having a concentration of 180 micrograms of skatole per gram of physiological saline solution according to the Response Factor Test disclosed herein.

6. A sensor capable of selectively detecting the presence of fecal material according to Claim 1 wherein said sensor device comprises microorganisms.

7. A sensor capable of selectively detecting the presence of fecal material according to Claim 6 wherein said microorganisms are immobilized on a substrate selected from the group of: polymer based materials, hydrogels, tissues, nonwoven materials, woven materials.

8. A sensor capable of selectively detecting the presence of fecal material according to Claim 6 wherein said microorganisms are selected from the group of: *Acinetobacter baumannii TOI63, Bacillus sp TOI41.*

9. A hygienic article comprising a sensor according to any of the preceding claims.
